# FASCICULE DE BREVET EUROPEEN

(11) **EP 1 483 013 B1**
(45) Date de publication et mention de la délivrance du brevet: **10.12.2008**
(21) Numéro de dépôt: 03735771.2
(22) Date de dépôt: 07.03.2003
(51) Int. Cl.: A61M 39/10, A61M 5/34, F16L 13/00, F16L 37/084

(54) **ENSEMBLE DE CONNEXION A USAGE MEDICAL POUR LE TRANSFERT DE FLUIDES**
VERBINDUNGSEINRICHTUNG ZUM MEDIZINISCHEN GEBRAUCH UND ZUM TRANSFER VON FLÜSSIGKEITEN
MEDICAL USAGE CONNECTOR ASSEMBLY FOR THE TRANSFER OF FLUIDS

(30) Priorité: 08.03.2002 FR 0202948
(43) Date de publication de la demande: 08.12.2004
(73) Titulaire: Eyegate Pharma SAS, 75012 Paris (FR)
(72) Inventeur: ROY, Pierre, F-75019 Paris (FR); KLEINSINGER, Alain, F-92200 Neuilly Sur Seine (FR)
(74) Mandataire: Texier, Christian
(86) Numéro de dépôt international: PCT/FR2003/000736
(87) Numéro de publication internationale: WO 2003/076001

(56) Documents cités:
- WO-A-99/44652
- DE-A- 19 614 684
- US-A- 2 922 419
- US-A- 3 491 757
- US-A- 5 868 433

## Description

L'invention concerne un système de connexions à usage médical adapté au transfert de fluide, notamment aux médicaments, depuis un réservoir vers un dispositif médical de délivrance. L'état de la technique le plus proche est le document US-A-5 868 433, qui définit les préambules des revendications 1 et 12.

Dans la suite, on entend par "fluide", les différents médicaments ou principes actifs obtenus par synthèse chimique ou composé d'extrait naturel, destinés à soigner ou traiter une infection ou une affection corporelle, voire de corriger ou de modifier une fonction organique, ainsi que d'établir un diagnostic. On peut citer, par exemple, des anti-inflammatoires, des antibiotiques, les antiviraux, les antifongiques, les anticancéreux, des antiangiogéniques, les antiglaucomateux, les neuroprotecteurs, les neuromodulateurs, les anesthésiques, les produits destinés à la thérapie génique comme les oligonucléotides, les plasmides, ainsi que les éléments nutritifs, les vitamines, les sels, les minéraux et les produits hormonaux. Un fluide peut aussi s'entendre comme étant l'un des fluides corporels comme le sang ou l'un de ses composés, les sucs gastriques, l'urine etc...

Pour plus de précision sur la définition d'un médicament, la Directive Européenne 65/65/CEE du 26 janvier 1965 concernant les dispositions législatives relatives aux médicaments en donne une définition :

« Pour l'application de la présente directive, il faut entendre par :
*1. Spécialité pharmaceutique :* tout médicament préparé à l'avance, mis sur le marché sous une dénomination spéciale et sous un conditionnement particulier.
*2. Médicament :* toute substance ou composition présentée comme possédant des propriétés curatives ou préventives à l'égard des maladies humaines ou animales.
   Toute substance ou composition pouvant être administrée à l'homme ou à l'animal en vue d'établir un diagnostic médical ou de restaurer, corriger ou modifier des fonctions organiques chez l'homme ou l'animal est également considérée comme médicament.
*3. Substance :* toute matière quelle qu'en soit l'origine, celle-ci pouvant être :
   - humaine, telle que : le sang humain et les produits dérivés du sang humain,
   - animale, telle que : les micro-organismes, animaux entiers, parties d'organes, sécrétions animales, toxines, substances obtenues par extraction, produits dérivés du sang, etc.
   - végétale, telle que : les micro-organismes, plantes, parties de plantes, sécrétions végétales, substances obtenues par extraction, etc.
   - chimique, telle que : les éléments, matières chimiques naturelles et les produits chimiques de transformation et de synthèse.
*4. Formule magistrale :* tout médicament préparé en pharmacie selon une prescription destinée à un malade déterminé.
*5. Formule officinale :* tout médicament préparé en pharmacie selon les indications d'une pharmacopée et destiné à être délivré directement aux patients approvisionnés par cette pharmacie. »

Cela est complété par la Directive 2309/93 du 22 juillet 1993:

« Médicaments issus de l'un des procédés biotechnologiques suivants:
- technologie de l'acide désoxyribonucléique recombinant,
- expression contrôlée de gènes codant pour des protéines biologiquement actives dans des procaryotes et des eucaryotes, y compris des cellules transformées de mammifères,
- méthodes à base d'hybridomes et d'anticorps monoclonaux.

Médicaments vétérinaires, y compris ceux non issus de la biotechnologie, destinés principalement à être utilisés comme améliorateurs de performance pour promouvoir la croissance ou pour augmenter la productivité des animaux traités. »

Actuellement, il est d'usage voire obligatoire, depuis la prise en considération des risques de contamination croisés entre patient ou entre patient et personnel soignant, que le dispositif de délivrance médicale soit utilisé une seule fois pour un patient donné, comme l'atteste le développement du matériel dit "à usage unique" vendu stérile et jeté après utilisation. De même, les médicaments sont de plus en plus fréquemment vendus dans des flacons contenant une seule dose, évitant qu'ils soient utilisés sur plusieurs patients et limitant les risques d'erreur de dosage. Par contre, malgré l'indication "usage unique" signalée par les dispositifs de délivrance médicaux, rien n'empêche que ceux-ci soient effectivement réutilisés sur plusieurs patients. Ainsi, par exemple, une aiguille pour injection intraveineuse peut être connectée à une seringue contenant une dose de médicament et donc être utilisé pour délivrer ladite dose de médicament sur plusieurs malades. En ce qui concerne ces aiguilles hypodermiques et ces seringues, de nombreuses réponses à la problématique de leur réutilisation, d'une part, et de la contamination accidentelle par piqûre, d'autre part, ont été proposées et conduisent invariablement à une utilisation unique de la seringue et/ou de l'aiguille.

Cependant, certains médicaments se présentent sous une forme destinée à être préparés sous dilution, une poudre lyophilisée par exemple. La préparation d'un tel médicament consiste à mélanger la forme à diluer avec un solvant selon certaines proportions et à transférer la solution obtenue dans le dispositif médical de délivrance. De manière générale, le solvant est prélevé une première fois avec la seringue puis injecté dans le récipient comprenant la poudre lyophilisée. Une fois le mélange effectué, on prélève de nouveau avec la seringue le mélange. Puis la seringue est connectée au dispositif médical de délivrance pour une administration dudit mélange. De ce fait, la connexion entre la seringue et les différents récipients doit pouvoir être mise en place et désassemblée aussi souvent que nécessaire, de manière à réaliser le mélange. La dernière connexion effectuée est celle de la seringue au dispositif médical de délivrance.

Le brevet US 6 231 552 décrit un ensemble de connexion entre un réservoir, ici une seringue, et un dispositif médical de délivrance, ici une aiguille hypodermique. Cet ensemble de connexion présente au niveau de la seringue un cône Luer standard mâle entouré par un cylindre coaxial au cône présentant sur sa face interne un filetage à l'extrémité libre duquel est aménagée une encoche. L'aiguille présente un cône Luer femelle standard compatible avec le cône Luer mâle de la seringue ainsi qu'un filetage complémentaire du filetage de la seringue aménagé sur la surface externe de la base de l'aiguille hypodermique. De plus, un ergot s'entend en saillie de ladite surface externe au niveau de l'extrémité distale du filetage de l'aiguille. Ainsi, lors de la connexion de la seringue à l'aiguille, en fin de vissage pour faire coopérer les deux filetages sus mentionnés, l'ergot de l'aiguille hypodermique vient se clipper de manière irréversible dans l'encoche de la seringue prévue à cet effet. Ceci permet de générer un système empêchant la déconnexion entre le réservoir et le dispositif médical de délivrance tout en permettant la connexion des Luer mâle et femelle de type "lock" (présentant un filetage tel que décrit précédemment) standards de manière réversible. Le système devient irréversible que dans le cas de la connexion de Luer aménagés comprenant soit le plot en saillie soit l'encoche apte à recevoir ledit plot.

L'inconvénient d'un tel dispositif est que l'utilisateur, pour monter de manière irréversible l'ensemble de connexion d'un tel système, doit effectuer un mouvement de translation associé à un mouvement de rotation (mouvement hélicoïdal) pour réaliser la connexion tout en fournissant un effort supplémentaire en fin de vissage pour enclencher lesdits moyens de retenue irréversible entre les deux éléments alors que les deux cônes Luer mâle et femelle coopèrent déjà par contact de manière à réaliser l'étanchéité de la connexion.

Un but de l'invention est de fournir un ensemble de connexion irréversible entre un réservoir et un dispositif de délivrance médicale s'assemblant lors d'un mouvement de connexion extrêmement simple.

Pour réaliser ce but, on prévoit, selon l'invention, un ensemble de connexion pour le transfert de fluide, notamment de fluide contenant des principes actifs, depuis un réservoir vers un dispositif médical de délivrance, comme défini par la revendication 1.

Ainsi, cet agencement particulier des moyens de retenue permet à l'utilisateur reliant un dispositif médical de délivrance à un réservoir, tous deux équipés de tels éléments de connexion, de les connecter l'un à l'autre de manière irréversible en effectuant un simple mouvement de translation, comme il le fait habituellement avec des éléments de connexion standard, comme des cônes Luer mâle et femelle.

Avantageusement, l'ensemble de connexion présente au moins l'une des caractéristiques supplémentaires suivantes :
- l'un parmi les premiers et deuxièmes moyens de retenue comporte au moins une languette déformable élastiquement et l'autre parmi les premiers et deuxièmes moyens de retenue comportent au moins une lèvre apte à coopérer avec ladite languette,
- l'un parmi les premiers et deuxièmes moyens de retenue comporte deux languettes situées de part et d'autre de l'élément de connexion,
- l'un parmi les premiers et deuxièmes moyens de retenue comporte au moins un évidemment et l'autre parmi les premiers et deuxièmes moyens de retenue comportent au moins une lèvre apte à coopérer avec l'évidement,
- la lèvre est déformable élastiquement,
- l'un parmi les premiers et deuxièmes éléments de connexion comporte une partie mâle et l'autre une partie femelle complémentaire de forme de la partie mâle et apte à coopérer avec cette dernière de sorte à rendre étanche la connexion,
- les parties mâle et femelle sont des cônes Luer à 6% environ, et
- la partie mâle est un perforateur de forme essentiellement tubulaire.

On prévoit aussi selon l'invention un élément de connexion pour le transfert de fluide, notamment de fluide contenant des principes actifs, depuis un réservoir vers un dispositif médical de délivrance, comme défini par la revendication 12.

D'autres caractéristiques et avantages de l'invention découleront de la description ci-après d'un mode de réalisation et de variantes. Aux dessins annexés :
- les figures 1a, 1b et 1c sont des vues en coupe ou en trois dimensions d'un premier mode de réalisation de l'invention en position déconnectée puis connectée,
- les figures 2a, 2b et 2c sont des vues en coupe ou en trois dimensions d'une variante de réalisation du mode de réalisation de la figure 1,
- la figure 3 est une vue en trois dimensions d'une seconde variante de réalisation d'un élément de connexion de la figure 1,
- la figure 4 est une vue en coupe d'une troisième variante de réalisation de l'ensemble de connexion de la figure 1,
- la figure 5 est une illustration d'un premier mode d'utilisation de l'invention de la figure 1,
- la figure 6 est une illustration d'un second mode d'utilisation de l'invention de la figure 1,
- la figure 7 et un troisième mode d'utilisation de l'invention de la figure 1,
- la figure 8 est une illustration d'un mode d'utilisation de l'invention selon la figure 2 dans le cadre de produits lyophilisés,
- la figure 9 et une vue en coupe d'un second mode de réalisation de l'invention, et
- la figure 10 est une illustration d'un mode d'utilisation de l'invention de la figure 9.

Dans la suite de la description, nous entendons par :
- Dispositif médical de délivrance, un dispositif destiné à entrer en contact avec un tissu du corps humain (peau, muqueuse, muscle, etc) ou à pénétrer dans une des cavités du corps humain (cavité orale, nasale, vessie, parties génitales, oeil, poumon etc) ou à pénétrer dans un système circulatoire (veine, artère) de sorte à permettre l'administration systémique ou locale d'un principe actif thérapeutique ou le prélèvement d'un fluide corporel, c'est-à-dire un fluide tel que défini plus haut. On peut donner comme exemple les dispositifs suivants considérés comme un dispositif médical de délivrance : patch transdermique, système de vaccination intramusculaire, transdermique, cathéter intraveineux, sonde urétrale, sonde gastrique, aiguille hypodermique, système de délivrance de médicament par iontophorèse, par électroporation ou autre source d'énergie, cathéter de prélèvement bronchique, etc.,
- Réservoir de médicaments ou de fluide. Un réservoir contenant le médicament prêt à être administré. Il peut s'agir d'un flacon souple ou rigide unidose, d'un flacon contenant un traitement destiné à être administré plusieurs jours soit de façon continue (poche souple de perfusion par exemple), soit discontinue (traitement antibiotique par exemple), d'une seringue contenant la dose de médicament après préparation, d'un flacon aérosol, etc. Ce réservoir de médicament est toujours muni d'un embout permettant la connexion sur le dispositif médical, comme un cône Luer standard pour une seringue ou un opercule en caoutchouc perforable pour un flacon rigide en verre, par exemple.
- Luer, un élément de connexion constitué d'un cône de pente 6% environ, mâle ou femelle et d'un diamètre d'entrée de 4mm environ (pour le mâle) et de 4,30mm environ (pour le femelle), standardisé, utilisé dans le domaine médical, défini par des normes internationales ISO et équipant la totalité des lignes de perfusion et de transfusion (cathéter intraveineux, seringue et aiguille hypodermique...)
- Percuteur, une aiguille creuse en métal ou en plastique utilisée pour perforer les opercules en caoutchouc (septa) des conteneurs de médicaments (vials) et avoir accès au contenu. Il n'y a pas de standard particulier sur ces percuteurs seulement sur les vials.

L'ensemble de connexion 1 comprend deux éléments de connexion 10,20 aptes à coopérer l'un avec l'autre de manière à réaliser une connexion, d'une part, irréversible et, d'autre part, étanche.

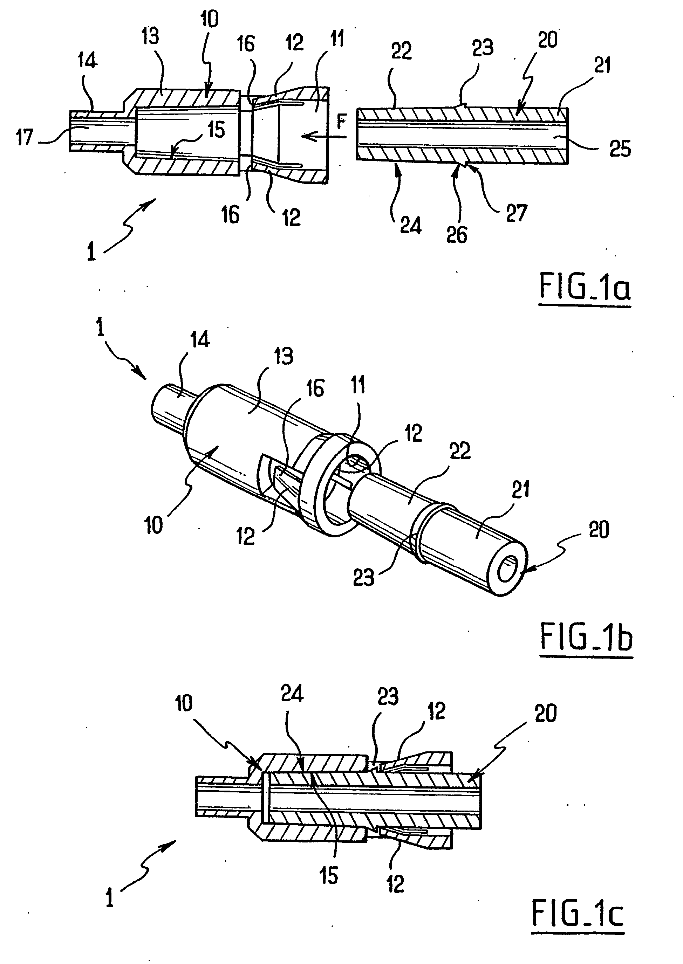

L'élément de connexion 10, appelé aussi connecteur femelle, est de forme générale allongée et de révolution. Il est traversé, de part en part et coaxialement à l'axe de révolution (non représenté) par un conduit interne 17. Le connecteur femelle 10 comporte trois parties fonctionnelles. La première partie 14 est préférentiellement une zone d'interface avec le dispositif médical de délivrance. Cette partie permet la fixation du connecteur femelle sur le dispositif médical de délivrance (non représenté). Par exemple, dans le cas illustré à la figure 1, cette zone est de forme essentiellement tubulaire, apte à recevoir une aiguille ou un tube qui est assemblé, de manière préférentielle, par collage ou par surmoulage. Cette zone d'interface 14 peut faire entièrement partie du dispositif médical de délivrance. Dans ce cas, le connecteur femelle 10 fait partie du dispositif médical de délivrance et est intégrée à celui-ci.

La deuxième partie fonctionnelle du connecteur femelle est une zone d'étanchéité 13 de forme externe généralement tubulaire et présentant une face interne 15 de contact ici sensiblement équivalent à un cône Luer femelle tel que précédemment défini et délimitant une partie du conduit interne 17.

La troisième partie du connecteur femelle est une zone 11 destinée à collaborer avec l'autre élément de connexion pour réaliser une connexion irréversible.

La zone 11 du connecteur femelle 10 comporte une couronne et une série de languettes 12 venues de matière avec la couronne. Ces languettes présentent une extrémité libre 16 s'étendant dans le conduit intérieur 17 et dirigée dans le sens d'introduction du connecteur mâle 20 dans le connecteur femelle 10 illustré par la flèche F. Ces languettes 12 sont déformables élastiquement.

L'élément de connexion 20, ou connecteur mâle, est de forme générale allongée et de révolution. Il est traversé, de part en part et coaxialement à l'axe de révolution (non représenté) par un conduit interne 25. Le connecteur mâle 20 présente, de la même manière, trois parties fonctionnelles comme suit. La partie 21 est une zone d'interface avec le réservoir contenant le médicament. De la même manière que précédemment, cette zone d'interface 21 fait partie du réservoir de médicaments de manière préférentielle. Ici, elle se présente sous la forme d'une zone essentiellement tubulaire quelconque. De manière préférentielle, cette zone d'interface 21 peut faire entièrement partie d'un réservoir de médicaments. Ainsi, le connecteur mâle 20 fait partie du réservoir et est intégré de ce fait à celui-ci. Par exemple, comme on le verra ultérieurement, il est possible d'équiper un flacon souple unidose de médicaments avec un tel connecteur mâle.

Une deuxième partie 23 est destinée à collaborer avec la zone 11 du connecteur femelle de façon à réaliser la connexion irréversible. La partie 23 comprend une lèvre 23 s'étendant en saillie depuis la surface externe du connecteur mâle. La lèvre 23 est préférentiellement continue sur toute la circonférence du connecteur mâle 20, comme illustré en figure 1b. La lèvre 23 présente une première face 26 inclinée selon un angle compris préférentiellement entre 10° et 45° environ avec l'axe de révolution du connecteur mâle, puis une deuxième face 27 perpendiculaire sensiblement à l'axe de révolution. L'intersection des faces 26, 27 formant un sommet de la lèvre 23. La face 26 est dirigée dans le sens d'introduction illustré par la flèche F, et la face 27, à l'opposée.

Enfin, la troisième partie formant le connecteur mâle est une zone d'étanchéité 22 présentant une surface externe 24 sensiblement équivalente à un cône Luer mâle, tel que précédemment défini.

L'assemblage du connecteur femelle 10 avec le connecteur mâle 20 se fait selon un mouvement unique de translation dans le sens de la flèche F. Lors de l'introduction du connecteur mâle dans le connecteur femelle, la lèvre 23 va déformer de manière élastique les languettes 12, la face 26 repoussant leur extrémité 16, puis une fois le sommet de la lèvre 23 passé, les extrémités 16 des languettes 12 reprennent leur position initiale empêchant dès ce moment la déconnexion entre les deux éléments de connexion. En effet, si on essaie de réaliser un mouvement inverse à la translation selon la flèche F, les extrémités 16 des languettes 12 viennent en butée contre la lèvre 23, en particulier contre la face 27, bloquant ainsi le mouvement. Une fois le connecteur mâle 20 introduit dans le connecteur femelle 10, la zone d'étanchéité 22 vient coopérer avec la zone d'étanchéité 13 par un contact entre le cône Luer femelle 15 et le cône Luer mâle 24, qui, du fait de leur complémentarité de forme, réalise ladite étanchéité de la connexion. Ainsi, le fluide passant par le conduit interne 25 du connecteur mâle peut circuler ensuite dans le conduit interne 17 du connecteur femelle vers le dispositif médical de délivrance sans qu'il n'y ait de fuite du fluide vers l'extérieur au niveau de l'ensemble de connexion. L'assemblage réalisé est illustré en figure 1c.

Il est à noter que le fait que la partie 22 formant zone d'étanchéité du connecteur mâle 20 soit sensiblement équivalente à un cône Luer femelle permet de connecter le réservoir de médicament sur toute autre dispositif comprenant un connecteur femelle de type Luer standard. Cela permet de réaliser les solutions à partir de produits lyophilisés et de solvants qui nécessitent la connexion avec le dispositif contenant le solvant puis une connexion avec le dispositif comprenant le produit lyophilisé de manière à réaliser la solution avant de se connecter sur le dispositif médical de délivrance.

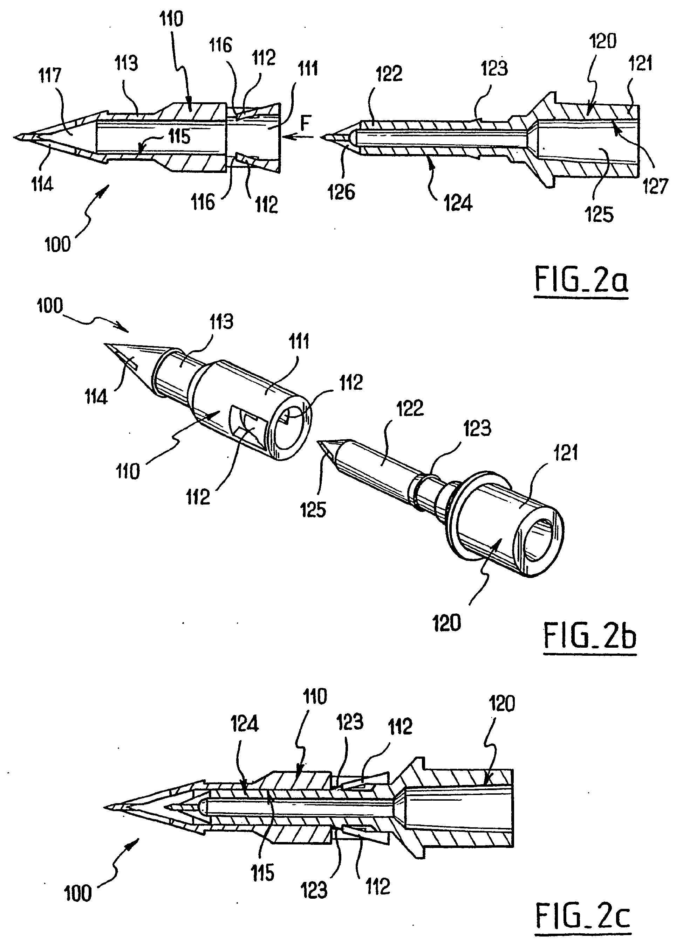

En référence aux figures 2a à 2c, nous allons décrire une variante du mode de réalisation précédent. L'ensemble de connexion 100 comporte un élément de connexion 110 ou connecteur femelle ainsi qu'un élément de connexion 120 ou connecteur mâle. De même que précédemment chacun des connecteurs est de révolution et de forme allongée. Il comprend un conduit interne 117, 125 coaxial à l'axe de révolution (non représenté). Il présente trois parties fonctionnelles. Le connecteur femelle 110 présente une partie fonctionnelle 114 destinée à réaliser l'interface avec le dispositif de médicament ici se présentant sous la forme d'une pointe conique formant embout destiné à être inséré en force dans un tube souple relié au dispositif médical de délivrance. De manière préférentielle, comme dans le cas précédent la zone d'interface 114 fait partie entièrement de dispositif médical de délivrance et ainsi le connecteur médical 110 fait partie intégrante du dispositif médical de délivrance. Le connecteur femelle 110 présente une deuxième partie 113 apte à réaliser la zone d'étanchéité présentant une face interne de contact 115 de forme essentiellement tubulaire qui est apte à coopérer avec une forme complémentaire du connecteur mâle 120 tel que nous allons le décrire ci-dessous. Enfin, le connecteur femelle 110 présente une troisième partie 111 très similaire à la partie 11 du mode de réalisation précédente comportant une couronne 111 ainsi que deux languettes 112 opposées l'une à l'autre de part et d'autre du conduit interne 117 . Les languettes comme précédemment sont venues de matière avec la couronne du connecteur 110 et présente une extrémité 116 libre dirigée dans le sens de l'introduction illustrée par la flèche F, et aussi dirigée vers l'intérieur du conduit 117.

Le connecteur mâle 120 présente un conduit interne 125 permettant de faire passer le fluide d'un réservoir à médicaments non représenté vers son extrémité opposée 126. Ce connecteur mâle 120 présente trois parties fonctionnelles. La partie 121 est destinée à réaliser l'interface avec un réservoir de médicament. Cette interface se réalise de manière identique au mode de réalisation précédent. Il est possible que cette interface comprenne un cône femelle de type Luer 127 destiné à coopérer avec une seringue standard présentant un cône Luer mâle complémentaire et formant le réservoir. Ensuite, le connecteur mâle 120 présente une partie formant zone d'étanchéité 122 comprenant une surface externe 124 essentiellement tubulaire apte à coopérer avec la surface 115 du connecteur femelle 110 de manière à réaliser une étanchéité. Enfin, le connecteur mâle 120 présente une zone 123 comprenant une lèvre 123 faisant saillie vers l'extérieur destinée à coopérer avec les languettes 112 du connecteur femelle 110 afin de réaliser la connexion irréversible. L'assemblage illustré en figure 2c est réalisé et se déroule de la même manière que l'assemblage du mode de réalisation précédente illustré en figure 1c et décrit précédemment.

Il est à noter que dans les figures 2a à 2c, la zone 122 du connecteur mâle 120 prend avantageusement la forme d'un percuteur apte à perforer les opercules en caoutchouc des flacons de médicaments ou vials. On peut imaginer par exemple que ce type de connecteur soit monté ou fasse partie intégrante d'une seringue et permette de prélever de l'eau stérile, ou tout autre solvant pour injection, contenue dans un flacon operculé. Puis à transférer cette eau stérile dans un second flacon operculé contenant un lyophilisat de principes actifs afin de le diluer et d'en obtenir une solution, puis de réaliser une connexion irréversible définitive avec un dispositif médical de délivrance comportant un connecteur femelle de 110 décrit précédemment.

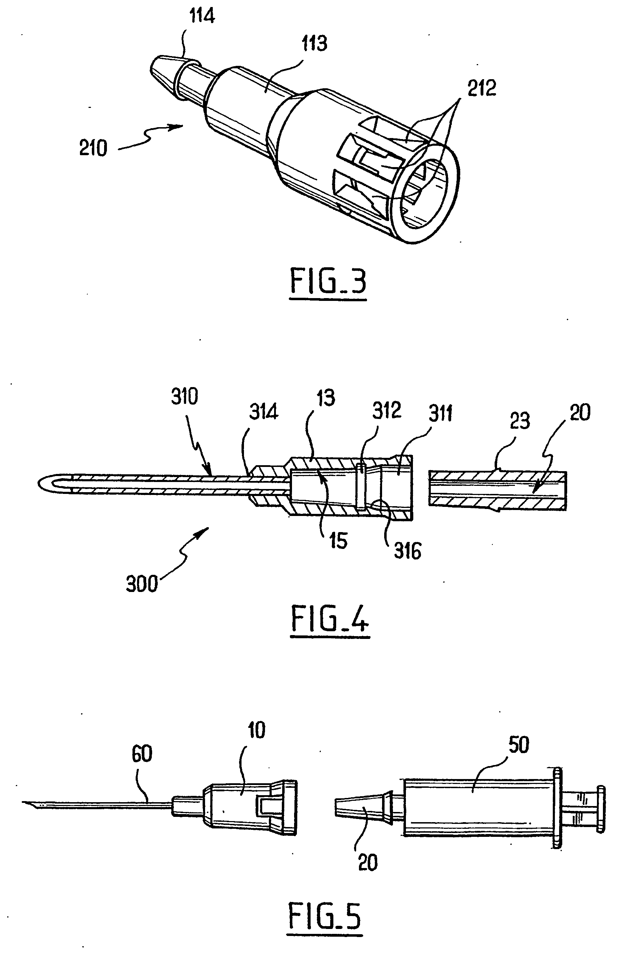

Le nombre minimal de languettes 12 ou 112 est de 1. Cependant, il est très avantageux d'aménager un nombre pair de languettes réparties de manière uniforme sur la couronne du connecteur femelle. Une variante de réalisation est illustrée en figure 3 pour lequel un connecteur femelle 210 se différencie du connecteur femelle 110 par le fait qu'il comporte 8 languettes 212 uniformément réparties sur la couronne du connecteur 210. Industriellement, un nombre de languettes de 2, comme illustré en figures la à 2c, est préférable facilitant la réalisation du connecteur femelle par injection plastique.

Illustrée en figure 4, une deuxième variante de réalisation d'un connecteur femelle est de réaliser un évidement continu en forme de couronne 312. Ici, l'ensemble de connexion 300 comporte un connecteur mâle 10 tel que précédemment décrit et un connecteur femelle 310 associé à une aiguille 314 et présentant une structure identique au connecteur 10 précédemment décrit. Ce connecteur 310 se différencie en outre du connecteur 10 par le fait que la zone 311 ne présente pas de languette mais un évidement en forme de couronne 312 apte à recevoir la lèvre 23 du connecteur mâle 20 lors de l'assemblage. Une telle réalisation nécessite que le matériau réalisant le connecteur 310 soit un matériau élastique permettant la déformation d'un cône 316, situé entre l'évidement et l'entrée de connecteur, par la lèvre 23 de manière à laisser passer cette dernière jusqu'à ce qu'elle atteigne l'évidement 312.

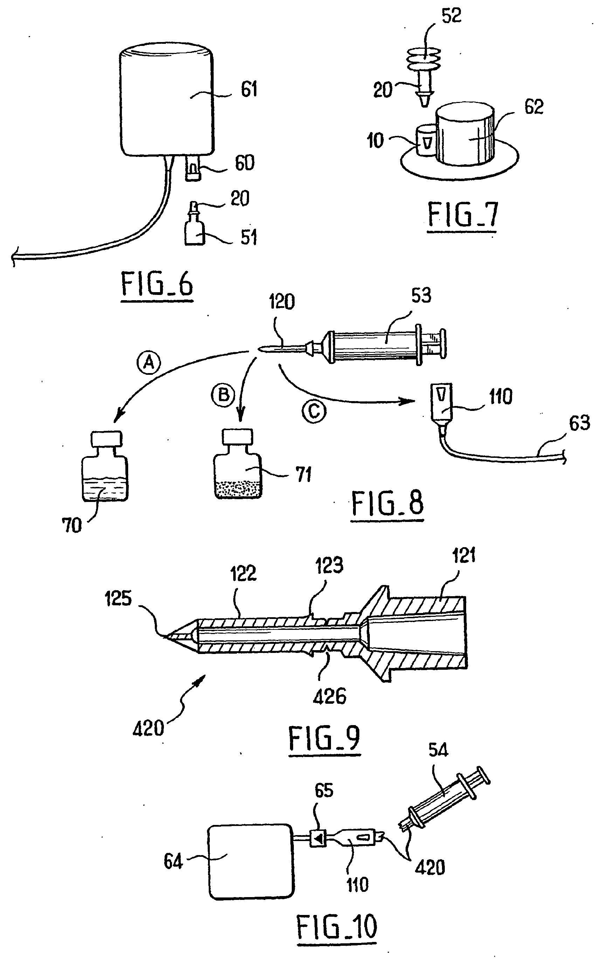

En référence aux figures 9 et 10, nous allons décrire une variante de réalisation du connecteur mâle 120 et un mode de réalisation de l'invention. Le connecteur mâle 420 est identique au connecteur mâle 120 si ce n'est qu'il présente sur toute sa circonférence externe une encoche 426 continue située entre la lèvre 123 et la partie 121 formant zone d'interface avec le réservoir de médicaments. Cette encoche 426 fragilise le connecteur mâle 420 à cet endroit de manière que, sous un léger effort de cisaillement, le connecteur se rompe à cet endroit. Cette partie dite sécable peut permettre, une fois le transfert de fluide effectué entre le réservoir de médicaments et le dispositif médical de délivrance de désolidariser ledit réservoir dudit dispositif en laissant une partie du connecteur mâle dans le connecteur femelle. Ceci permet de désolidariser la partie du réservoir de médicament du dispositif médical de délivrance, une fois le médicament transféré, afin de ne pas "alourdir" inutilement ledit dispositif médical de délivrance durant son utilisation. De plus, le fait de laisser une partie du connecteur mâle dans le connecteur femelle renforce l'interdiction de toute réutilisation ultérieure du connecteur femelle, donc du dispositif médical de délivrance. Une telle situation est illustré en figure 10 où un connecteur femelle 110 est lié à un dispositif médical de délivrance 64 par l'intermédiaire d'une valve anti-retour 65, ou anti-reflux, qui empêche le fluide de ressortir après injection et rupture du connecteur mâle. Le connecteur mâle 420 est ici représenté cassé au niveau de l'encoche 426 et fait partie intégrante d'une seringue formant réservoir de médicaments 54.

Il est à noter que de manière générale les connecteurs mâles et femelles précédemment décrits composant l'ensemble de connexion sont de préférence entièrement intégrés respectivement au réservoir de médicaments et au dispositif médical de délivrance.

En figure 5 est illustré un premier mode d'utilisation d'un ensemble de connexion illustré aux figures 1a à 1c, le connecteur femelle 10 est ici monté de façon solidaire à une aiguille hypodermique 60 qui pourrait être aussi un cathéter ou une sonde. Le connecteur mâle est quant à lui monté également de façon solidaire sur une seringue unidose ou non contenant le médicament. Il est important de noter que dans ce type de configuration, la seringue peut être connectée de façon réversible avec un Luer femelle standard. Par exemple, de ce fait, il est possible de prélever un médicament ou un fluide corporel d'une source équipée d'un connecteur Luer femelle standard, de se déconnecter une fois le médicament ou le fluide prélevé puis d'injecter ce fluide dans l'aiguille ou le cathéter susnommé sans pouvoir cette fois-ci se déconnecter à nouveau du dispositif médical de délivrance 60.

En figure 6, est illustré un deuxième mode d'utilisation de l'ensemble de connexion des figures 1a à 1c, pour lequel le dispositif médical de délivrance est une poche de perfusion préremplie de soluté (comme de l'eau glucosée par exemple) et présentant de manière intégrée un connecteur femelle 10 destiné à mélanger au soluté de la poche 61 une dose unique de médicament comme un antibiotique par exemple. Un flacon unidose 51 formant réservoir de médicaments et comportant de manière intégrée un connecteur mâle 20 peut être installé de manière irréversible dans le connecteur femelle 10. Le fait de placer ce type de connecteur sur la voie d'accès à la poche de perfusion rend alors impossible la réutilisation de cet accès et évite, de cette manière simple, tout risque de surdosage accidentel du médicament à injecter.

En figure 7 est illustré un troisième mode d'utilisation de l'ensemble de connexion de la figure la à 1c. Dans cet exemple, le dispositif médical de délivrance est un dispositif de transfert de médicaments de type intraoculaire ou transdermique ou transmuqueux, pour lequel le connecteur femelle 10 fait partie intégrante du dispositif. Un dispositif de transfert de médicament intraoculaire est décrit plus en détail dans la demande de brevet français FR 2 773 320. De fait, un flacon unidose 52 souple est équipé d'un connecteur 20. Ce flacon unidose, une fois connecté, au dispositif médical de délivrance et une fois le médicament transféré du flacon dans le dispositif médical de délivrance rend l'ensemble inutilisable une seconde fois.

En figure 8, est illustré un mode d'utilisation de l'invention illustrée aux figures 2a à 2c.

Est illustré dans cette figure, une procédure à suivre pour la préparation d'un médicament se présentant sous forme lyophilisée. Le connecteur mâle sous forme de percuteur 120 est monté de façon solidaire sur une seringue ou tout autre réservoir de médicaments muni d'un dispositif d'aspiration. Dans un premier temps A, on procède à l'aspiration de la quantité nécessaire de solution en perforant le septum du flacon 70 contenant celui-ci. Puis, dans une étape B, on injecte la solution, précédemment aspirée, dans un flacon 71 contenant un lyophilisat en perforant de la même manière le septum du flacon 71. Puis, on réaspire la solution de ce flacon. Dans une étape C, on connecte de manière irréversible la seringue 53 dans un connecteur femelle 110 relié au dispositif médical de délivrance 63 illustré ici simplement par un cordon d'alimentation.

Les matériaux utilisés pour la réalisation de ce type de connecteur sont de préférence des matériaux polymères couramment utilisés dans la fabrication des connecteurs des dispositifs à usage médical, possédant naturellement les propriétés élastiques permettant le clippage par déformation des languettes des connecteurs tout en offrant une bonne résistance mécanique et susceptible d'être moulés par injection de manière simple ou par coulée dans un moule de forme. Il s'agit par exemple de matériaux de la famille des polystyrènes, des polycarbonates des polychlorures de vinyle, des polyéthylènes, des polypropylènes, des polyuréthannes, des polyamides, des polysulfones etc..., cette liste n'étant pas exhaustive. De plus, certains alliages métalliques peuvent convenir à cette application, l'aptitude à la déformation élastique réversible étant un critère de choix de tels alliages.

Il est à noter, d'autre part, que, dans les modes de réalisations illustrés dans les figures, le connecteur femelle de ces modes n'est pas utilisable avec des connecteurs Luer mâle standard.

Bien entendu, on pourra apporter à l'invention de nombreuses modifications sans sortir du cadre de celle-ci.

Par exemple, le connecteur mâle présente, en lieu et place de la lèvre 23, des languettes déformables présentant une extrémité libre dirigée dans le sens d'introduction et vers l'extérieur apte à se déformer. Le connecteur femelle quant à lui présente, en lieu et place des languettes 12, une lèvre s'étendant en saillie à l'intérieur du conduit 17 et apte à coopérer avec les languettes du connecteur mâle pour rendre la connexion irréversible, la lèvre lors de l'introduction déformant les languettes qui après passage reprennent leurs positions initiales. Tout mouvement inverse pour déconnecter l'ensemble étant rendu impossible par le fait que lesdites languettes viennent en butée sur la lèvre.

## Revendications

1. Ensemble de connexion (10; 100; 300) pour le transfert de fluide, notamment de fluide contenant des principes actifs depuis un réservoir vers un dispositif médical de délivrance comportant :
un premier élément de connexion (20; 120) associé au réservoir et comprenant des premiers moyens de retenue (23; 123),
un deuxième élément de connexion (10; 110; 210; 310) associé au dispositif médical de délivrance et comprenant des deuxièmes moyens de retenue (12; 112; 212; 312) aptes à coopérer avec les premiers moyens de retenue pour former une connexion irréversible entre les deux éléments,
les premiers et deuxièmes moyens de retenue sont agencés de sorte qu'ils coopèrent l'un avec l'autre par clippage lors d'un mouvement de translation unique d'un élément de connexion par rapport à l'autre pour réaliser la connexion irréversible, **caractérisé en ce qu'**au moins l'un des éléments de connexion comporte des moyens (426) aptes à le rendre sécable, de sorte à pouvoir désolidariser ledit réservoir dudit dispositif médical.

2. Ensemble de connexion selon la revendication 1, **caractérisé en ce que** l'un parmi les premiers et deuxièmes moyens de retenue comporte au moins une languette (12; 112; 212) déformable élastiquement et l'autre parmi les premiers et deuxièmes moyens de retenue comporte au moins une lèvre (23; 123) apte a coopérer avec la languette.

3. Ensemble de connexion selon la revendication 2, **caractérisé en ce que** l'un parmi des premiers et deuxièmes moyens de retenue comporte deux languettes situées de part et d'autre de l'élément de connexion.

4. Ensemble de connexion selon la revendication 1, **caractérisé en ce que** l'un parmi des premiers et deuxièmes moyens de retenue comporte au moins un évidement (312) et l'autre parmi les premiers et deuxièmes moyens de retenue comporte au moins une lèvre apte à coopérer avec l'évidement.

5. Ensemble de connexion selon l'une des revendications 1 ou 4, **caractérisé en ce que** la lèvre est déformable élastiquement.

6. Ensemble de connexion selon l'une des revendications 1 à 5, **caractérisé en ce que** l'un parmi les premiers et deuxièmes éléments de connexion comporte une partie mâle (22; 122) et l'autre une partie femelle (13; 113) complémentaire de forme de la partie mâle et apte à coopérer avec cette dernière de sorte à rendre la connexion étanche aux fuites.

7. Ensemble de connexion selon la revendication 6, **caractérisé en ce que** les parties mâles et femelles sont des cônes Luer (15, 24) de pente à 6% environ.

8. Ensemble de connexion selon la revendication 6, **caractérisé en ce que** la partie mâle est un perforateur de forme essentiellement tubulaire.

9. Ensemble de connexion selon l'une des revendications 1 à 8, **caractérisé en ce qu'**il comprend en outre des moyens anti-retour aptes à empêcher le fluide de ressortir après injection et mise en oeuvre des moyens aptes à rendre l'élément de connexion sécable.

10. Ensemble de connexion selon la revendication 7, **caractérisé en ce qu'**au moins l'un parmi les premier et deuxième éléments de connexion est apte à se connecter de façon réversible sur des connecteurs standards préalablement à la réalisation d'une connexion irréversible avec l'autre.

11. Ensemble de connexion selon la revendication 8, **caractérisé en ce que** le perforateur est apte a permettre à un parmi les premier et deuxième éléments de connexion d'effectuer des connexions réversibles sur des récipients préalablement à la réalisation d'une connexion irréversible avec l'autre.

12. Elément de connexion pour le transfert de fluide, notamment de fluide contenant des principes actifs, depuis un réservoir vers un dispositif médical de délivrance, comprenant des moyens de retenue, les moyens de retenue étant agencés de sorte qu'ils coopèrent avec des moyens de retenue d'un autre élément de connexion par clippage lors d'un mouvement de translation unique de l'élément de connexion par rapport à l'autre pour réaliser une connexion irréversible entre les deux éléments de connexion, **caractérisé en ce que** l'élément de connexion comporte des moyens aptes à le rendre sécable de sorte à pouvoir désolidariser ledit réservoir dudit dispositif médical.

## Claims

1. Connection assembly (10; 100; 300) for transfer of fluid, particularly fluid containing active constituents, from a reservoir to a medical delivery device comprising:
a first connection element (20; 120) associated with the reservoir and comprising first retaining means (23; 123),
a second connection element (10; 110; 210; 310) associated with the medical delivery device and comprising second retaining means (12; 112; 212; 312) capable of cooperating with the first retaining means to form an irreversible connection between the two elements,
the first and second retaining means are arranged such that they cooperate with each other by clipping during a single translation movement of one connection element with respect to the other to make the connection irreversible, **characterised in that** at least one of the connection elements comprises means (426) capable of making it breakable, so as to be able to detach said reservoir from said medical device.

2. Connection assembly according to claim 1, **characterised in that** either the first or second retaining means comprises at least one elastically deformable tab (12; 112; 212) and the other retaining means comprises at least one lip (23; 123) capable of cooperating with the tab.

3. Connection assembly according to claim 2, **characterised in that** either the first or second retaining means comprises two tabs located on opposite sides of the connection element.

4. Connection assembly according to claim 1, **characterised in that** either the first or second retaining means comprises at least one recess (312) and the other retaining means comprises at least one lip capable of cooperating with the recess.

5. Connection assembly according either claim 1 or 4, **characterised in that** the lip is elastically deformable.

6. Connection assembly according to any of claims 1 to 5, **characterised in that** either the first or second connection element comprises a male part (22; 22) and the other connection element comprises a female part (13; 113) with a shape complementary to the male part and capable of cooperating with the male part so as to make the connection leak tight.

7. Connection assembly according to claim 6, **characterised in that** the male and female parts are Luer cones (15, 24) with a gradient of approximately 6%.

8. Connection assembly according to claim 6, **characterised in that** the male part is an essentially tubular shaped perforator.

9. Connection assembly according to any of claims 1 to 8, **characterised in that** it also comprises non-return means capable of preventing fluid from coming out after injection and use of means capable of making the connection element breakable.

10. Connection assembly according to claim 7, **characterised in that** either the first or second connection element can be irreversibly connected to standard connectors before an irreversible connection is made with the other element.

11. Connection assembly according to claim 8, **characterised in that** the perforator is capable of enabling either the first or second connection element to make reversible connections on receptacles before an irreversible connection is made with the other element.

12. Connection element for transfer of fluid, particularly fluid containing active constituents, from a reservoir to a medical delivery device, comprising retaining means, the retaining means being arranged such that they cooperate with retaining means in another connection element by clipping during a single translation movement between the two elements to make an irreversible connection between the two connection elements, **characterised in that** the connection element comprises means capable of making it breakable, so as to be able to detach said reservoir from said medical device.

## Patentansprüche

1. Verbindungseinheit (10; 100; 300) für die Übertragung einer Flüssigkeit, insbesondere einer Flüssigkeit, die Wirkstoffe enthält, von einem Vorratsbehälter zu einer medizinischen Vorrichtung zum Verabreichen, folgendes umfassend:
ein erstes Verbindungselement (20; 120), das mit dem Vorratsbehälter verbunden ist und das erste Mittel zum Festhalten (23; 123) umfasst,
ein zweites Verbindungselement (10; 110; 210; 310), das mit der medizinischen Vorrichtung zum Verabreichen verbunden ist und das zweite Mittel zum Festhalten (12; 112; 212; 312) umfasst, die dafür geeignet sind, mit den ersten Mitteln zum Festhalten zusammenzuarbeiten, um eine irreversible Verbindung zwischen den beiden Elementen zu bilden,
wobei die ersten und zweiten Mittel zum Festhalten derart eingerichtet sind, dass sie untereinander durch einen Klippvorgang zusammenarbeiten, und dies durch eine alleinige Verschiebungsbewegung eines Verbindungselements relativ zum anderen zur Herstellung der irreversiblen Verbindung, **dadurch gekennzeichnet, dass** wenigstens eines der Verbindungselemente Mittel (426) umfasst, die dazu geeignet sind, es teilbar zu machen, derart, dass der Vorratsbehälter von der medizinischen Vorrichtung abgetrennt werden kann.

2. Verbindungseinheit nach Anspruch 1, **dadurch gekennzeichnet, dass** eines von den ersten und den zweiten Festhaltemitteln wenigstens eine elastisch verformbare Zunge (12; 112; 212) umfasst, und das andere von den ersten und den zweiten Festhaltemitteln wenigstens eine hervorspringende Kante (23; 123) umfasst, die dazu geeignet ist, mit der Zunge zusammenzuarbeiten.

3. Verbindungseinheit nach Anspruch 2, **dadurch gekennzeichnet, dass** eines von den ersten und den zweiten Festhaltemitteln zwei Zungen umfasst, die sich auf beiden Seiten des Verbindungselements befinden.

4. Verbindungseinheit nach Anspruch 1, **dadurch gekennzeichnet, dass** eines von den ersten und den zweiten Festhaltemitteln wenigstens eine Aussparung (312) umfasst und das andere von den ersten und den zweiten Festhaltemitteln wenigstens eine hervorspringende Kante umfasst, die dazu geeignet ist, mit der Aussparung zusammenzuarbeiten.

5. Verbindungseinheit nach einem der Ansprüche 1 bis 4, **dadurch gekennzeichnet, dass** die hervorspringende Kante elastisch verformbar ist.

6. Verbindungseinheit nach einem der Ansprüche 1 bis 5, **dadurch gekennzeichnet, dass** eines von den ersten und den zweiten Verbindungselementen einen männlichen Abschnitt (22; 122) umfasst und das andere einen weiblichen Abschnitt (13; 113) umfasst, der formkomplementär zum männlichen Abschnitt ist und der dazu geeignet ist, mit letzterem derart zusammenzuarbeiten, dass die Verbindung gegenüber Leckverlusten dicht ist.

7. Verbindungseinheit nach Anspruch 6, **dadurch gekennzeichnet, dass** die männlichen und weiblichen Abschnitte Luer-Kegel (15, 24) mit einer Schräge von etwa 6% sind.

8. Verbindungseinheit nach Anspruch 6, **dadurch gekennzeichnet, dass** der männliche Abschnitt eine im wesentlichen röhrenförmige Lochungsvorrichtung ist.

9. Verbindungseinheit nach einem der Ansprüche 1 bis 8, **dadurch gekennzeichnet, dass** sie außerdem Rücklaufschutzmittel umfasst, die dazu geeignet sind, die Flüssigkeit daran zu hindern, nach der Injektion und dem Einsatz der Mittel, die dazu geeignet sind, das Verbindungselement teilbar zu machen, wieder auszutreten.

10. Verbindungseinheit nach Anspruch 7, **dadurch gekennzeichnet, dass** wenigstens eines von den ersten und den zweiten Verbindungselementen dazu geeignet ist, vor der Herstellung einer irreversiblen Verbindung mit dem anderen, reversibel an Standardanschlüsse anschließbar zu sein.

11. Verbindungseinheit nach Anspruch 8, **dadurch gekennzeichnet, dass** die Lochungsvorrichtung dazu geeignet ist, es einem von den ersten und den zweiten Verbindungselementen zu gestatten, vor der Herstellung einer irreversiblen Verbindung mit dem anderen, reversible Verbindungen mit Behältnissen einzugehen.

12. Verbindungselement für die Übertragung von Flüssigkeiten, insbesondere von Flüssigkeiten, die Wirkstoffe enthalten, von einem Vorratsbehälter zu einer medizinischen Vorrichtung zum Verabreichen, das Mittel zum Festhalten umfasst, wobei die Festhaltemittel derart eingerichtet sind, dass sie mit Festhaltemitteln eines anderen Verbindungselements durch einen Klippvorgang zusammenwirken, und dies durch eine alleinige Verschiebungsbewegung des Verbindungselements relativ zum anderen, um eine irreversible Verbindung zwischen den beiden Verbindungselementen herzustellen, **dadurch gekennzeichnet, dass** das Verbindungselement Mittel umfasst, die dazu geeignet sind, es teilbar zu machen, derart, dass der Vorratsbehälter von der medizinischen Vorrichtung abgetrennt werden kann.
